# EUROPÄISCHE PATENTANMELDUNG

(11) **EP 1 990 054 A1**
(43) Veröffentlichungstag der Anmeldung: **12.11.2008**
(21) Anmeldenummer: 07090098.0
(22) Anmeldetag: 11.05.2007
(51) Int. Cl.: A61K 31/52, A61K 31/522, A61K 31/513, A61P 35/04

(54) **Polymerase-Hemmer und ihre Verwendung zur Behandlung von Tumoren**

(71) Anmelder: Freie Universität Berlin, 14195 Berlin (DE)
(72) Erfinder: Schäfer-Korting, Monika, 14195 Berlin (DE); Höltje, Hans-Dieter, 10119 Berlin (DE)
(74) Vertreter: Boeckh, Tobias

(57) **Zusammenfassung**

Die Erfindung betrifft Polymerase-Hemmer, insbesondere Polymerase alpha-Hemmer und ihre Verwendung bei Zellwachstumsstörungen, insbesondere Tumorerkrankungen, bevorzugt Basaliome und/oder spinozelluläre Karzinome.

## Beschreibung

Die Erfindung betrifft Polymerase-Hemmer, insbesondere Polymerase alpha-Hemmer **[Gibt es eine "übliche Schreibeweise"? Die Verwendung von "α" ist nachteilig.]** und ihre Verwendung bei Zellwachstumsstörungen, insbesondere Tumorerkrankungen, bevorzugt Basaliome und/oder spinozelluläre Karzinome.

Tumorerkrankungen werden im Stand der Technik häufig synonym zu Krebs verwendet. Hierunter werden insbesondere bösartige Tumoren verstanden. Im engeren Sinne werden aber auch bösartige Hämoblastosen in Fachkreisen als Krebs definiert (z. B. Leukämie als Blutkrebs). Der Begriff Tumor umfasst neben dem o. g. Begriff des Krebs auch jede umschriebene Gewebsvermehrung bzw. Raumforderung in oder an einem humanen oder tierischen Körper. Dies beinhaltet sowohl die Schwellung bei einer Entzündung als auch Neubildungen von Körpergewebe durch Fehlregulation des Zellwachstums. Auch gutartige Tumore wie Muttermale oder Fettgeschwülste können als Krebs definiert werden, auch wenn dies nicht durchgängig in der Fachliteratur erfolgt. Krebs wird als Sammelbegriff für eine Vielzahl verwandter Krankheiten verwendet, bei denen Körperzellen unkontrolliert wachsen, sich teilen und gesundes Gewebe verdrängen bzw. flankieren oder dieses infiltrieren oder zerstören können.

Im Stand der Technik sind zahlreiche Mittel und Verfahren zur Behandlung von Tumorerkrankungen bekannt. Da Tumorerkrankungen zu den häufigsten Todesursachen gehören, muss aber davon ausgegangen werden, dass zahlreiche dieser Mittel und Verfahren nur bedingt erfolgreich angewendet werden können. Zudem ist bekannt, dass die genannten Mittel und Verfahren zahlreiche Nebenwirkungen aufweisen. Es sind im Stand der Technik Antikrebsmittel offenbart worden, deren Nebenwirkungen für den Patienten bedauerlicherweise deutlicher wahrnehmbar sind als die Hauptwirkung.

Bösartige Hauttumoren, auch als maligne Tumoren oder Hautkrebs bzw. malignes Melanom bezeichnet, gehören zu den häufigsten Tumorerkrankungen beim Menschen. Häufig entstehen diese auf Hautstellen, die der Sonnenstrahlung ausgesetzt sind, wie z. B. den Waden bei Radfahrern. Ganz allgemein sind häufig Menschen betroffen, die sich viel im Freien aufhalten, wie z. B. Bauern, Seeleute oder Fischer. Da spezifische Schichten der Bevölkerung braune Haut mit Erholung und Dynamik gleichsetzen, hat es in den vergangenen Jahren einen signifikanten Anstieg der Krebserkrankungen gegeben. Insbesondere in Ländern mit hoher Sonneneinstrahlung wie z. B. Australien ist es zu einer alarmierenden Häufung von Hautkrebserkrankungen gekommen. Aber auch in Deutschland erkranken ca. 8.000 Menschen an einer besonders bösartigen Form des Hautkrebs, dem malignen Melanom. Es kann bei den malignen Hauttumoren zwischen dem Basaliom (Basalzellkarzinom), dem Spinaliom (Spinalzellkarzinom) und dem malignen Melanom (schwarzer Hautkrebs) unterschieden werden. Das Mittel der Wahl bei Basaliom und Spinaliom ist das chirurgische Entfernen des Tumors. Das maligne Melanom gehört zu den Tumoren, die am frühesten metastasieren. Bei der Therapie dieses Tumors müssen daher auch die Tochtergeschwülste chirurgisch entfernt werden. Nach der Operation wird häufig eine so genannte Immuntherapie durchgeführt. Insbesondere mit Hilfe von Interferon sollen die körpereigenen Abwehrkräfte gestärkt werden. Eine Chemotherapie mit Zytostatika wird heute nur noch sehr begrenzt als einzige Therapie eingesetzt. Dies hat seine Ursache auch darin, dass mit derartigen Mitteln die genannten Tumoren nicht lokal begrenzt behandelt werden können.

Im Stand der Technik sind weiterhin Polymerase-Hemmer bekannt, die die Reduplikation der DNA bzw. RNA inhibieren und so beispielsweise dem Wachstum von Viren oder entarteten Zellen entgegenwirken können. Auch wenn die Wirkung dieser so genannten Nukleosidanaloga in der Literatur umfassend diskutiert wird, sind die praktischen Erfolge bescheiden und bleiben regelmäßig hinter den Erwartungen zurück. **[Es ist erforderlich, dass wir einige Nachteile des "Standes der Technik" benennen.]** Auch wenn die Verwendung derartiger Strukturen in der Virus- oder Krebstherapie naheliegend erscheint, ist der Fachmann durch den widersprüchlichen Stand der Technik und die zahlreichen negativen Ergebnisse beim praktischen Einsatz dieser Strukturen keinesfalls motiviert, Nukleoside/Nukleosid-Analoga zur Krebstherapie einzusetzen. Insbesondere hat sich gezeigt, dass bereits geringe Änderungen der Struktur der Nukleoside zu erheblichen Aktivitätseinbußen oder zu einer drastischen Zunahme der Nebenwirkungen führt. Derartige Probleme sind mit Hilfe von Routineversuchen, bei denen neue Strukturen aufgefunden werden sollen, nicht behebbar, da die Zahl der möglichen einzusetzenden Strukturen nahezu unendlich ist.

Aufgabe der Erfindung war es daher, insbesondere konkrete ausgewählte Nukleoside bereitzustellen, die bei ausgewählten Tumoren eine überraschend gute Wirkung haben.

Gelöst wird diese Aufgabe durch die Bereitstellung eines Mittels zur Behandlung von Zellmodifikationen in einem Hautareal, insbesondere ausgewählt aus der Gruppe umfassend aktinische Keratose, maligne Melanome, spinozelluläre Karzinome und/oder Basaliome, das eine Polymerase moduliert und das gegenüber Diclophenac und/oder 5-Fu eine verbesserte antiproliferative Aktivität aufweisen, wobei sie ausgewählt sind aus der Gruppe umfassend Verbindungen gemäß den allgemeinen Formeln 1 bis 4:

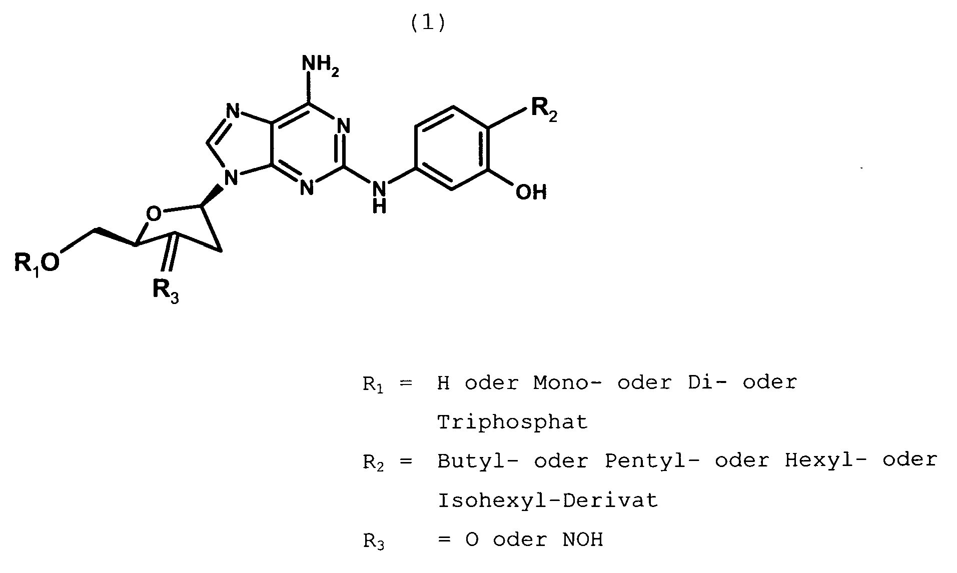

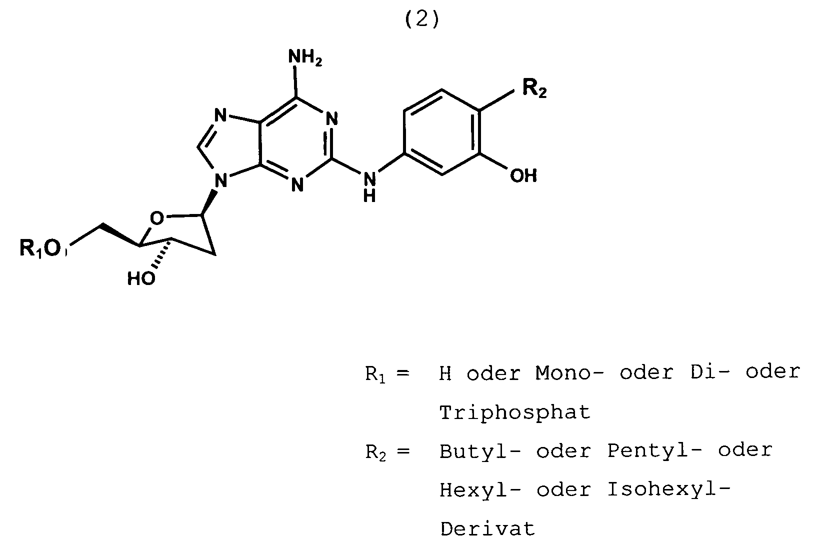

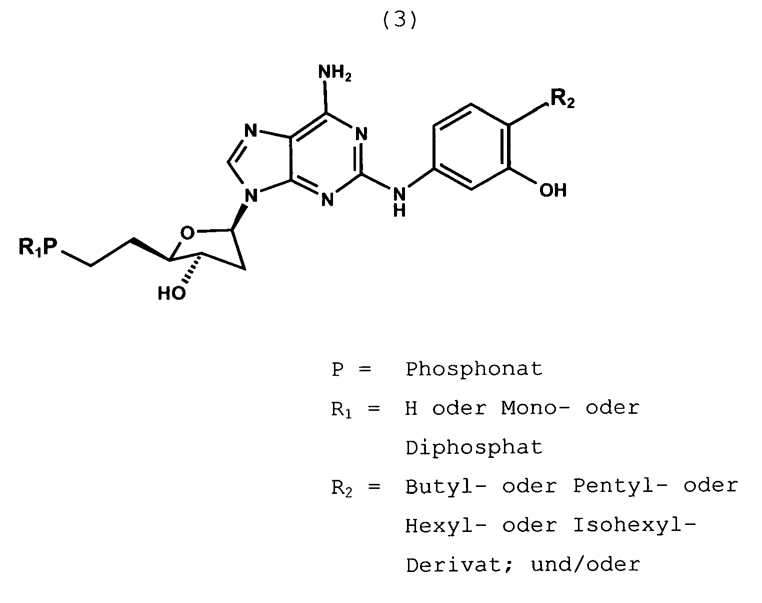

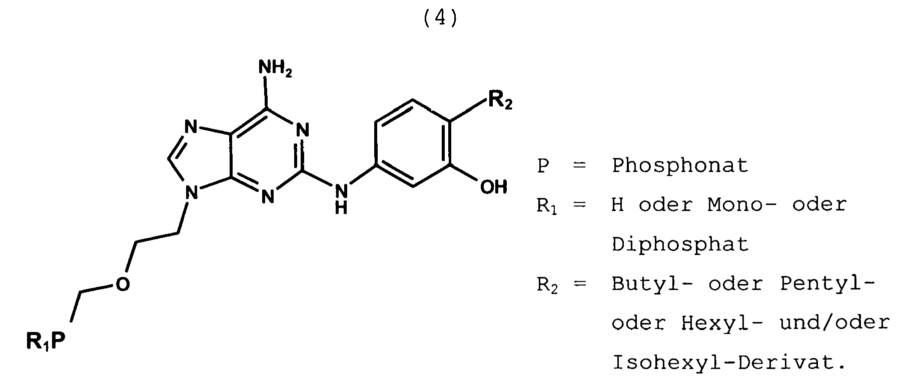

Es war völlig überraschend, dass die genannten Verbindungen gemäß der allgemeinen Formeln 1 bis 4 sehr gut eingesetzt werden können, um Zellmodifikationen in einem Hautareal zu behandeln. Ein Hautareal im Sinne der Erfindung ist jedes Areal, welches mit der Epidermis, der Dermis, dem Subcutis oder Hautanhangsgebilden, der Leistenhaut bzw. der Felderhaut im Zusammenhang steht, diese ganz oder teilweise bildet oder als solche vorliegt, oder mit diesen assoziiert vorliegt. Im Sinne der Erfindung wird die Haut als äußeres Organ des menschlichen oder tierischen Organismus verstanden, welches der Abgrenzung von innen und außen dient. Zu einem Hautareal im Sinne der Erfindung gehören Bestandteile der Oberhaut, der Lederhaut oder der Unterhaut. Die Oberhaut (Epidermis) besteht erfindungsgemäß aus folgenden Schichten: Hornschicht (Stratum corneum), Leuchtschicht (Stratum lucidum), Körnerschicht (Stratum granulosum), Stachelzellschicht (Stratum spinosum) und/oder Basalschicht (Stratum basale). Jede Modifikation von Zellen in diesem Bereich ist eine Zellmodifikation in einem Hautareal im Sinne der Erfindung.

Die Lederhaut (Dermis, Corium), die ebenfalls ein Bestandteil der erfindungsgemäßen Hautareale sein kann, besteht vorwiegend aus Bindegewebsfasern und dient der Ernährung und Verankerung der Epidermis. Das kapillarisierte Blutgefäßsystem in der Grenzzone zur Epidermis gehört mit zum Hautareal im Sinne der Erfindung ebenso wie die Talg- und Schweißdrüsen. Die Dermis im Sinne der Erfindung kann in ein Stratum papillare und in ein Stratum reticulare unterteilt werden.

Weiterhin kann ein Hautareal im Sinne der Erfindung jedes Areal, d. h. jeder Ort in oder an der Unterhaut (Subcutis) sein.

Weiterhin werden durch den erfindungsgemäßen Begriff des Hautareals auch Hautanhanggebilde umfasst, wie Haare, Talgdrüsen, Haarbalgmuskeln, Nägeln, Hörner und Schweißdrüsen, insbesondere die ekkrinen und die apokrinen Schweißdrüsen, aber auch die Milchdrüsen. Jede Zellmodifikation, insbesondere ein vom normalen abweichendes Zellwachstum, kann mit den erfindungsgemäßen Mitteln behandelt werden.

Die Hautareale im Sinne der Erfindung können aber auch die Leistenhaut betreffen, wie sie an den Fingern oder an der Fußsohle auftreten oder aber die Felderhaut und die hiermit assoziierten Hautanhangsgebilde.

Auf allen genannten Hauttypen können sich vollständig oder teilweise Schwellungen oder Geschwülste bilden. D. h., es kann zu einer Zunahme des Gewebsvolumen jedweder Ursache kommen. Selbstverständlich ist es auch möglich, dass es zu einer Abnahme des Gewebsvolumens kommt. Diese Änderungen des Gewebsvolumens werden in einer Ausführungsform der Erfindung als Zellmodifikationen im Sinne der Erfindung verstanden. Aber auch Änderungen der Aktivität der Zellen, ohne dass es zu einer Zu- oder Abnahme des Gewebsvolumens kommt, können Zellmodifikationen im Sinne der Erfindung sein, wenn beispielsweise bestimmte modifizierte Zellen in ein anderes Zellgewebe, welches zu einem Hautareal gehören kann oder aber auch nicht, infiltrieren oder eindringen. Auch diese Prozesse werden als Zellmodifikationen im Sinne der Erfindung verstanden.

Auch eine Entzündung, welche ein Hautareal betrifft oder an dieses angrenzt, ist eine Zellmodifikation im Sinne der Erfindung.

Bevorzugte Zellmodifikationen in einem Hautareal sind die aktinische Keratose, maligne Melanome, spinozelluläre Karzinome oder Basaliome.

Die aktinische Keratose im Sinne der Erfindung ist eine insbesondere durch Lichtschädigung verursachte Veränderung der Haut, insbesondere der verhornten Oberhaut. Die aktinische Keratose kann bösartig entarten und zu Hautkrebs führen (fakultative Präkanzerose). Die aktinische Keratose betrifft Menschen in allen Lebensjahren, aber in einer bevorzugten Ausführungsform der Erfindung insbesondere solche in der zweiten Lebenshälfte. Die aktinische Keratose tritt in einer bevorzugten Ausführungsform der Erfindung insbesondere im Gesicht, auf dem Handrücken, der Stirn, im Oberkopf/Glatze, auf der Nase und dem Ohr auf. Die aktinische Keratose ist selbstverständlich nicht auf den Menschen beschränkt, sondern sie betrifft alle Lebewesen, insbesondere Säugetieren, besonders bevorzugt Haushunde und Hauskatzen. Es ist selbstverständlich möglich, dass die aktinische Keratose als Zellmodifikation in einem Hautareal mit anderen Hautveränderungen wie Krebsvorstufen assoziiert oder nicht assoziiert, zeitgleich oder zeitversetzt in einem Organismus vorkommt (z. B. Morbus Bowen).

Das Basaliom ist im Sinne der Erfindung ein Basalzellenkrebs bzw. Vorstufen von diesem Krebs, der auch als Epithelioma basocellulare bezeichnet werden kann. Insbesondere kann diese Zellmodifikation von den basalen Epidermiszellschichten ausgehen. Sie kann auch das umliegende Nichthautgewebe schädigen sowie die Knochenzellen infiltrieren. Jede Behandlung einer solchen Zellmodifikation wird als Behandlung einer Zellmodifikation eines Hautareals im Sinne der Erfindung verstanden, vor allem da die Zellmodifikation von einem Hautareal, nämlich den E-pidermiszellschichten, ausgeht.

Ein malignes Melanom im Sinne der Erfindung ist jede insbesondere bösartige (maligne) Entartung von Pigmentzellen (Melanozyten). Das maligne Melanom weist eine starke Tendenz auf, früh Metastasen über das Lymph- und Blutbahnensystem zu streuen. Demgemäß umfasst die Erfindung als eine Zellmodifikation in einem Hautareal auch die Behandlung von Metastasen, die von einem malignen Melanom ausgehen.

Ein spinozelluläres Karzinom im Sinne der Erfindung ist eine Zellmodifikation in einem Hautareal, die insbesondere durch UV-Licht ausgelöst wird und daher insbesondere an Körperstellen auftritt, die - ähnlich wie bei dem Basaliom - dem Licht ausgesetzt sind, wie beispielsweise das Gesicht. Präkanzerosen wie die aktinische Keratose und Morbus Bowen können in bevorzugten Ausführungsvarianten der Erfindung als Spinaliom aufgefasst werden. Auch die Metastasen, die vom Spinaliom oder beispielsweise vom Basaliom gebildet werden, werden im Sinne der Erfindung als Zellmodifikation in einem Hautareal verstanden.

Das erfindungsgemäße Mittel ist geeignet, insbesondere die Polymerase der Zellen, die modifiziert sind, indem sie verstärkt wachsen oder ihre biochemische Aktivität ändern, zu beeinflussen, insbesondere zu inhibieren. Die erfindungsgemäßen Mittel können daher im Zusammenhang mit der Beeinflussung, Reduplikation bzw. Reparatur von DNA oder RNA insbesondere in Tumoren eingesetzt werden. Überraschenderweise hat sich gezeigt, dass die erfindungsgemäßen Mittel insbesondere bei der Lokaltherapie von Tumoren eingesetzt werden, die in einem Hautareal auftreten, wobei es sich bei diesen Tumoren um Primär- oder Sekundärtumoren in dem Hautareal handeln kann. Ein bevorzugtes Target der erfindungsgemäßen Mittel sind demgemäß die Polymerasen, die im Tumorgewebe - d. h. insbesondere in den Zellen - vorliegen. Hierbei kann es sich innerhalb des Tumorgewebes oder des angrenzenden Gewebes auch um Zellen handeln, die nicht oder noch nicht modifiziert - beispielsweise entartet - vorliegen. Auch wenn es bereits Mittel gibt, die für ähnliche Tumoren verwendet werden können, wie beispielsweise Diclofenac und/oder 5-Fu (nach deren Effektivität gegen Tumorzellen diese im Stand der Technik klassifiziert werden können), so war es völlig überraschend, dass die erfindungsgemäßen Strukturen besonders effektiv zur Behandlung insbesondere von Hauttumoren eingesetzt werden können. Bevorzugt weisen die erfindungsgemäßen Mittel eine hohe Affinität zur Polymerase alpha auf, die so nicht mehr an eine Primase bindet, dass die Replikation des Haupt- und Folgestranges nicht erfolgen kann. Selbstverständlich können die erfindungsgemäßen Mittel gegen alle Polymerasen, insbesondere eukaryotische Polymerasen bevorzugt der Klassen A, B, X und Y eingesetzt werden. Überraschend ist aber insbesondere die Wirkung der Mittel auf die Polymerase alpha im Zusammenhang mit der Behandlung der genannten Zellmodifikationen. Die erfindungsgemäßen Mittel können daher auch als DNA-Polymerase-Hemmer bzw. -Inhibitor bezeichnet werden.

Neben dieser Verwendung können die erfindungsgemäßen Strukturen aber auch als Leitstruktur für die Entwicklung anderer Polymerase-Hemmer, insbesondere Polymerase-alpha-Hemmer eingesetzt werden. Die Erfindung ist aber selbstverständlich nicht nur auf die erfindungsgemäßen Mittel gemäß der allgemeinen Formeln 1 bis 4 beschränkt, sondern sie betrifft auch funktionsanaloge Moleküle, die im Vergleich zu den Molekülen gemäß der Formeln 1 bis 4 ein ähnliches Verhalten bei der Lösung des erfindungsgemäßen Problems zeigen. Funktionsanaloge im Sinne der Erfindung sind insbesondere als Äquivalente aufzufassen, die zwar verschieden generiert werden können, aber im wesentlichen dieselbe Funktion auf im wesentlichen demselben Weg erfüllen und im wesentlichen dasselbe Ergebnis hervorbringen wie die Verbindungen gemäß der allgemeinen Formeln 1 bis 4. Der Begriff der Funktionsanalogen im Zusammenhang mit der erfindungsgemäßen Lehre ist daher ausreichend klar, da ausgeschlossen wird, dass ein Mittel unter Schutz gestellt werden soll, dass über das zu erreichende Ergebnis definiert wird. Die Frage, ob Funktionsanaloge, d. h. Äquivalente von der erfindungsgemäßen Lehre erfasst werden, wird demnach insbesondere danach beantwortet, ob die Funktionsanalogen das der Erfindung zugrunde liegende Problem zwar mit abgewandelten aber objektiv gleichwirkenden Mitteln lösen und ob der Fachmann durch seine Fachkenntnis befähigt war, die abgewandelten Mittel (Funktionsanalogen bzw. Varianten) als gleichwirkend aufzufinden, wobei die Überlegung, die der Fachmann hierzu anstellen musste, sich derart am Sinngehalt der in den Patentansprüchen unter Schutz gestellten Lehre orientieren, dass der Fachmann die Verwendung der Funktionsanalogen und Varianten als eine Lösung der erfindungsgemäßen Aufgabe in Betracht gezogen hätte, die der Lösung der Aufgabe mit den Mitteln gemäß der allgemeinen Formeln 1 bis 4 gleichwertig ist. Anmeldungsgemäße funktionsanaloge Moleküle sind demgemäß nur solche, die der Fachmann als gleichwirkend auffinden würde, wobei der Fachmann hierzu Überlegungen anstellen muss, die sich insbesondere am Sinngehalt der im Patentanspruch unter Schutz gestellten Lehre orientieren, wobei der Fachmann die Funktionsanalogen oder Varianten als ein Lösung in Betracht zieht, die der Lösung gemäß den erfindungsgemäß beanspruchten Moleküle gemäß den allgemeinen Formeln 1 bis 4 gleichwertig ist.

Die Begriffe "Funktionsanaloge" oder "Varianten" sowie "im wesentlichen dieselbe Funktion", "auf im wesentlichen demselben Weg" und "im wesentlichen dasselbe Ergebnis" sind keine relativen Begriffe, da die betreffenden Ausdrücke auf dem betreffenden Fachgebiet der Biologie eine allgemein anerkannte Bedeutung haben. Da die Begriffe Funktionsanaloge und Varianten gemäß der Erfindung als Äquivalente verstanden werden und diese Begriffe durch die Rechtsprechung mehrfach definiert wurde (so unter anderem im Entwurf für den internationalen Patentrechtsharmonisierungsvertrag), sind die Begriffe auch ausreichend klar. Die Begriffe haben daher eine allgemein anerkannte Bedeutung, so dass sie nicht durch präzisere Angaben ersetzt werden müssen. Der Begriff "im wesentlichen" wird gemäß der Definition in dem internationalen Patentrechtsharmonisierungsvertrag zugelassen, weil er als so genannter "Weichzeichner" von zu scharf bzw. eng definierten Ansprüchen dient. Durch den Begriff "im wesentlichen" soll vermieden werden, dass ein Molekül, das eine Variante zu den erfindungsgemäß beanspruchten Molekülen ist und im wesentlichen dieselbe Funktion auf im wesentlichen demselben Weg mit im wesentlichen demselben Ergebnis hervorbringt, nicht mehr durch die Patentansprüche erfasst wird, wenn sie beispielsweise nicht exakt das identische Ergebnis hervorbringt oder wenn für den Einsatz derartige Funktionsanaloge oder Varianten leicht modifizierte Bedingungen für die optimale Verwendung erforderlich sind. Der Begriff "auf im wesentlichen demselben Weg" bedeutet daher im Sinne der Erfindung, dass die Funktionsanalogen, bzw. Varianten insbesondere für die Behandlung von aktinischen Keratosen, malignem Melanom, spinozellulären Karzinomen und/oder Basaliomen auf Hautarealen eingesetzt werden können.

In einer bevorzugtenn Ausführungsform der Erfindung weist das erfindungsgemäße Mittel folgende Struktur auf: 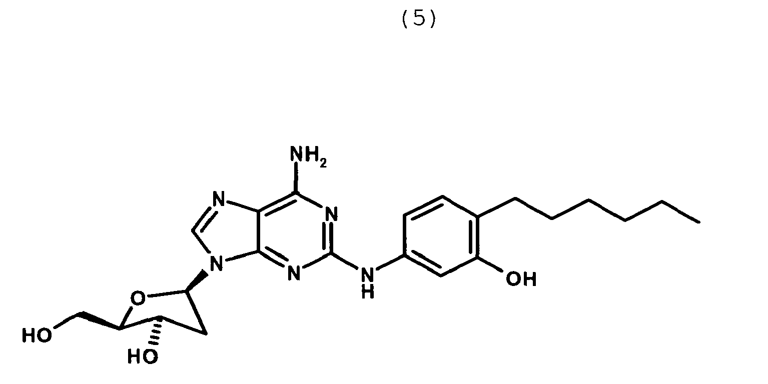 2-(4-Hexyl-3-hydroxyphenylamin) -2'-desoxyadenosin.

In einer weiteren bevorzugten Ausführungsform weist das erfindungsgemäße Mittel folgende Struktur auf:

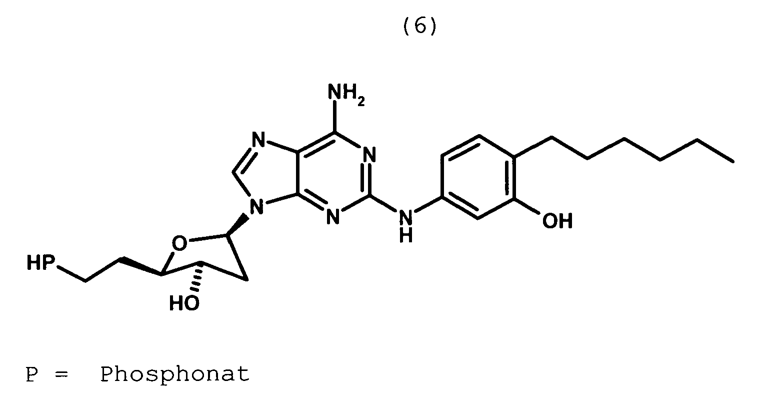

2-(4-Hexyl-3-hydroxyphenylamin)-9-[4-hydroxy-5-(2-phosphonoethyl)tetrahydro-2-furyl]adenin.

In einer weiteren bevorzugten Ausführungsform der Erfindung weist das erfindungsgemäße Mittel die folgende Struktur auf:

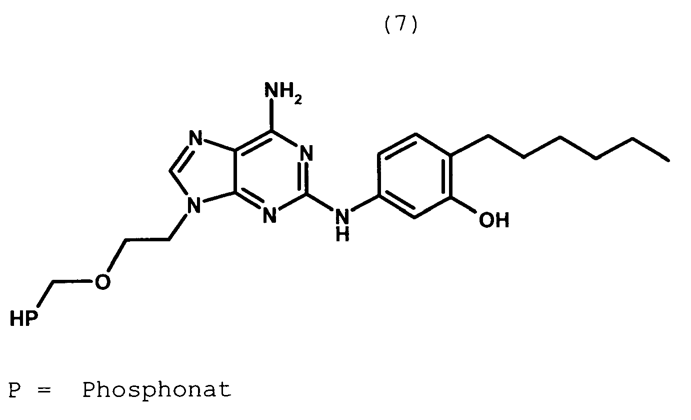

2-(4-Hexyl-3-hydroxyphenylamin)-9-[2-(phosphono-methoxy)ethyl]adenin.

Die Erfindung betrifft auch Mittel, die funktionsanalog zu den Verbindungen gemäß der allgemeinen Formeln 1 bis 7 sind und die ausgewählt sind aus der Gruppe umfassend 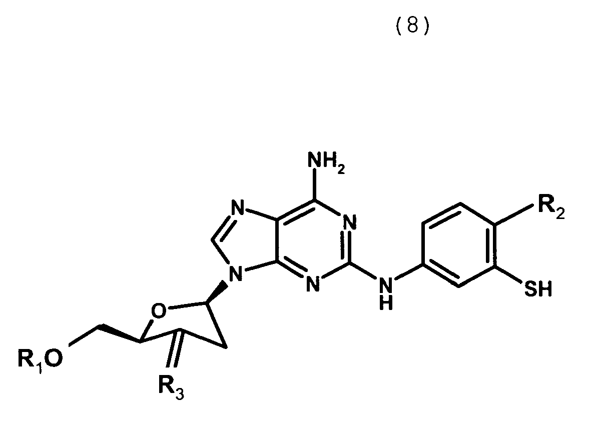 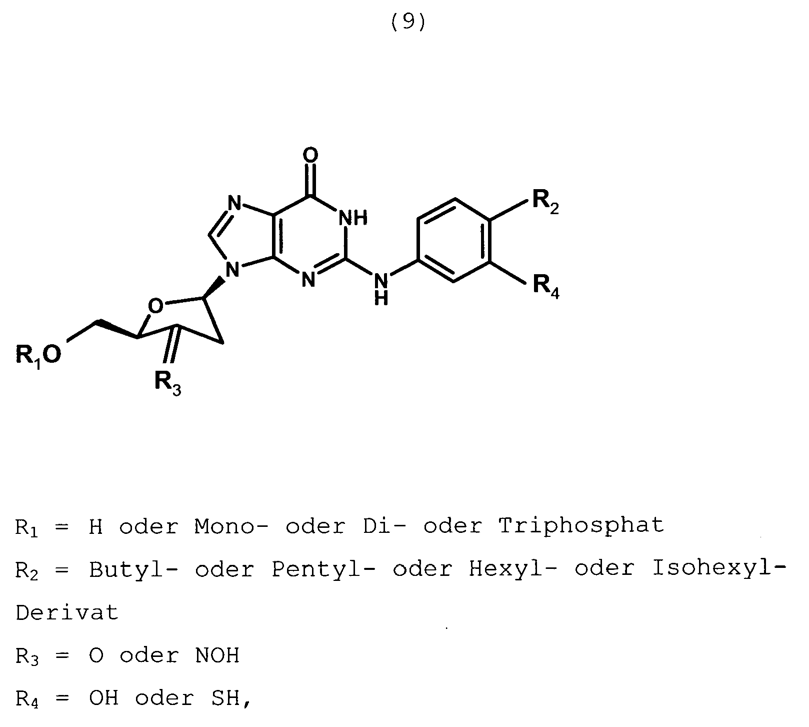 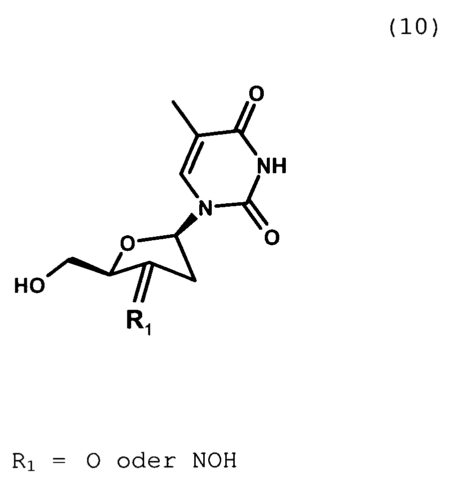 3'-Desoxy-3'-oxothymidin
bzw.
3'-Desoxy-3'-hydroxyliminothymidin,

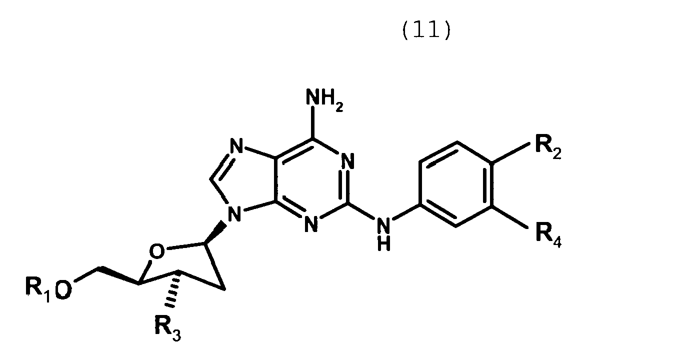

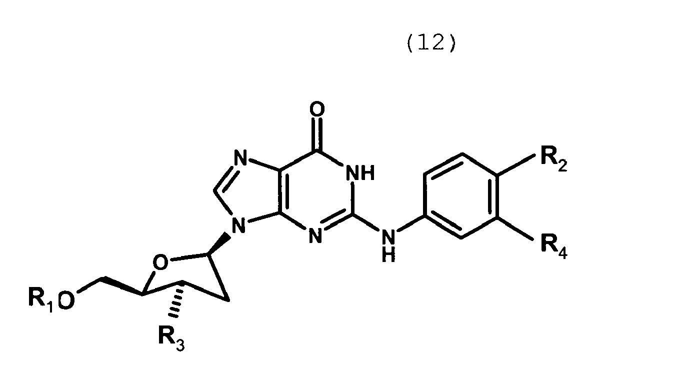

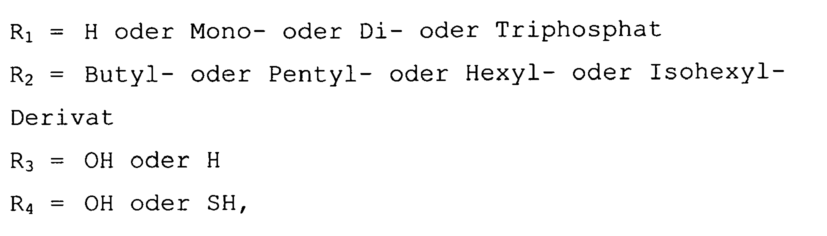

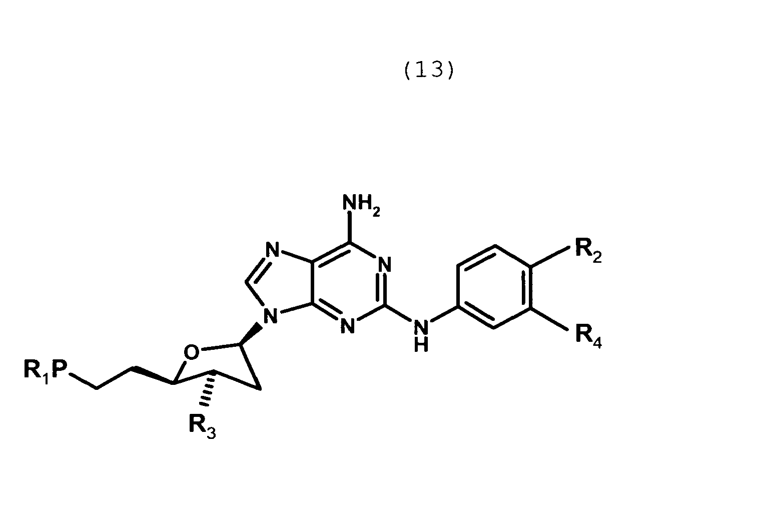

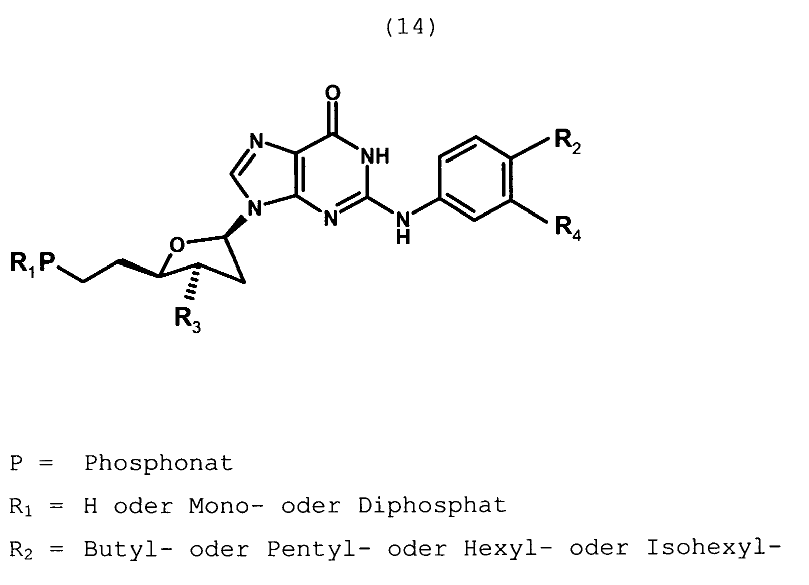

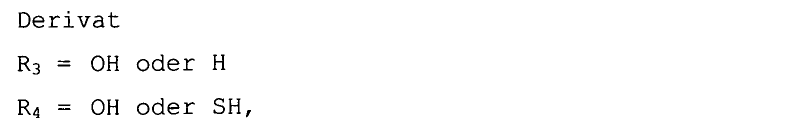

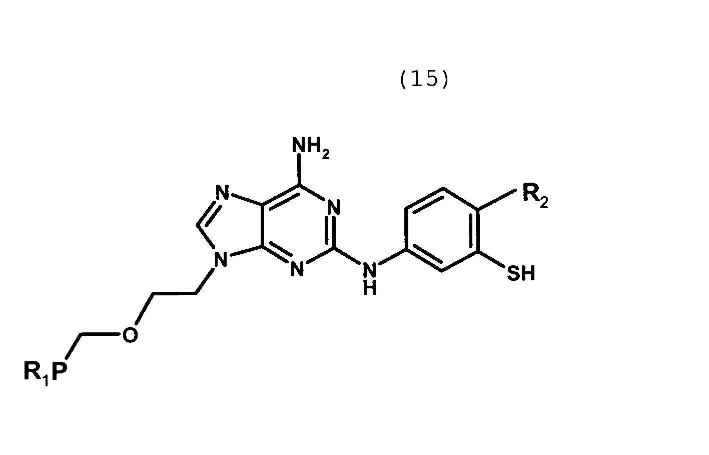

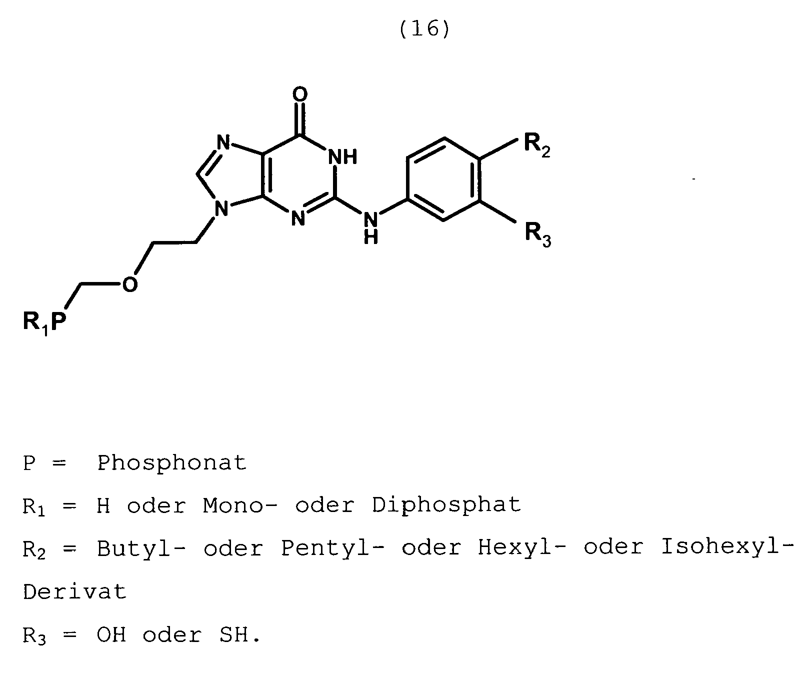

Die Erfindung betrifft demgemäß die beanspruchten Mittel gemäß der allgemeinen Formel 1 bis 16 für die Verwendung als Arzneimittel. Bevorzugt werden die Mittel als pharmazeutische Mittel eingesetzt, die das erfindungsgemäße Mittel umfassen und gegebenenfalls einen pharmazeutisch verträglichen Träger.

In einer bevorzugten Ausführungsform der Erfindung ist der pharmazeutische Träger ausgewählt aus der Gruppe umfassend Füllmittel, Streckmittel, Bindemittel, Feuchthaltemittel, Lösungsverzögerer, Sprengmittel, Resorptionsbeschleuniger, Netzmittel, Absorptionsmittel, Gleitmittel und/oder Trägerlipide, insbesondere Liposomen.

Die Erfindung betrifft auch einen Kit umfassend das erfindungsgemäße pharmazeutisches Mittel, zusammen mit einer Information zum Kombinieren der Inhalte des Kits.

In einem weiteren Aspekt betrifft die Erfindung die Verwendung der erfindungsgemäßen Mittel, der erfindungsgemä-βen pharmazeutischen Mittel sowie des erfindungsgemäßen Kits zur Modifikation einer Polymerase in vitro oder in vivo.

In einer bevorzugten Ausführungsform der Erfindung ist die die Polymerase eine DNA-Polymerase alpha.

In einer weiteren bevorzugten Ausführungsform der Erfindung ist die Modifikation der Polymerase die Modifikation des Zellwachstums und/oder einer Zellwachstumsstörung.

In einer weiteren bevorzugten Ausführungsform der Erfindung stellt die Zellwachstumsstörung eine Zellproliferation dar.

In einer weiteren bevorzugten Ausführungsform der Erfindung ist die Zellproliferation ausgewählt aus der Gruppe umfassend Hautkrebs mit Ursprung im Hautepithel, bevorzugt ein Basaliom oder Spinaliom, Hautkrebs der Pigmentzellen, Hautkrebs der Immunzellen, Fibrosarkome, Schweißdrüsenkarzinome, Talgdrüsenkarzinome, Angiosarkome, Myosarkome und/oder Merkelzellkarzinome.

In einer weiteren bevorzugten Ausführungsform der Erfindung ist das Basaliom knotiges, solides Basaliom, ein oberflächliches Basaliom, ein pigmentiertes Basaliom, ein sklerosierend wachsendes Basaliom, ein exulzierend wachsendes Basaliom und/oder ein destruierend wachsendes Basaliom.

In einer weiteren bevorzugten Ausführungsform der Erfindung ist die Zellwachstumsstörung eine Keratose.

In einer weiteren bevorzugten Ausführungsform der Erfindung ist die Keratose ausgewählt aus der Gruppe umfassend seborrhoische Keratose, aktinische Keratose, Altersflecken, Pigmentflecken und/oder Lentigo solaris.

In einer weiteren bevorzugten Ausführungsform der Erfindung ist die Tumorerkrankung ein Karzinom, ein Sarkom, ein neuroendokriner Tumor, ein hämoonkologischer Tumor, ein dysontogenetischer Tumor und/oder ein Mischtumor.

In einer weiteren bevorzugten Ausführungsform der Erfindung liegt das Mittel in Form einer Lösung, Emulsion, Suspension, Salbe, eines Balsams, Öls, Gels, Schaums, Augenbalsams, Augengels, Zäpfchens, Sprays, Pflasters, Sticks oder Stifts, in flüssiger Form, in Form von künstlichen Tränen, als thermoreversibles Gel (flüssig anzuwenden) und/oder in Form einer Creme vor.

In einer weiteren bevorzugten Ausführungsform der Erfindung wird das erfindungsgemäße pharmazeutische Mittel als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und angewendet

In einer weiteren bevorzugten Ausführungsform der Erfindung ist die Salbe ausgewählt ist aus der Gruppe umfassend Unguentum leniens (Kühlsalbe), Unguentum emulsificans (Hydrophile Salbe), Unguentum emulsificans aquosum (wasserhaltige hydrophile Salbe), Unguentum cetomacrogolis, Unguentum cetylicum cum aqua (Cetylsalbe), Unguentum Alcoholum Lanae bzw. Unguentum adeps lanae (Wollwachsalkoholsalbe, Eucerin) Unguentum molle (weiche Salbe) und/oder Unguentum Zinci (Zinksalbe).

In einer weiteren bevorzugten Ausführungsform der Erfindung liegt das erfindungsgemäße pharmazeutische Mittel in einer Konzentration von 0,1 bis 99,5, bevorzugt von 0,5 bis 95,0, besonders bevorzugt von 20,0 bis 80,0 Gew.-% in einer Zubereitung vor.

In einer weiteren bevorzugten Ausführungsform der Erfindung wird das erfindungsgemäße pharmazeutische Mittel in Gesamtmengen von 0,05 bis 500 mg pro kg, bevorzugt von 5 bis 100 mg pro kg Körpergewicht, je 24 Stunden eingesetzt.

In einer weiteren bevorzugten Ausführungsform der Erfindung wird die Zubereitung oral, subkutan, intravenös, intramuskulär, intraperitoneal und/oder topisch gesetzt.

In einer weiteren bevorzugten Ausführungsform der Erfindung werden die erfindungsgemäßen Mittel, die erfindungsgemäßen pharmazeutischen Mittel sowie der erfindungsgemä-βe Kit zur Sekundärprophylaxe von Tumoren eingesetzt.

Die Erfindung betrifft auch die Verwendung der erfindungsgemäßen Verbindungen bzw. der erfindungsgemäßen Moleküle gemäß der allgemeinen Formeln 1 bis 16 als Leitstruktur für die Entwicklung von Polymerase-Hemmern.

Im Folgenden soll die Erfindung anhand von Beispielen näher erläutert werden, ohne auf diese Beispiele beschränkt zu sein.

### Beispiel

HM-1 zeichnet sich durch eine starke zytotoxische Wirkung auf nicht kontaktgehemmte (Abb. 1) und kontaktgehemmte (Tab. 1) Tumorzellen (SCC-25-Zellen) aus. Dies ist stärker als bei primären Keratinozyten (Kc). Daraus resultiert eine selektive Schädigung des Hauttumors.

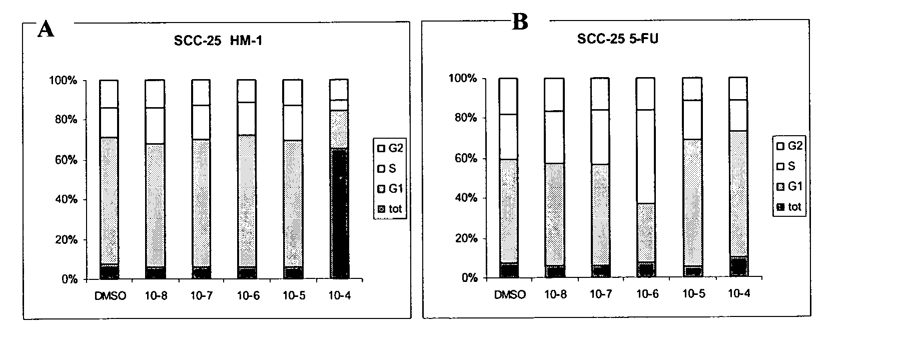

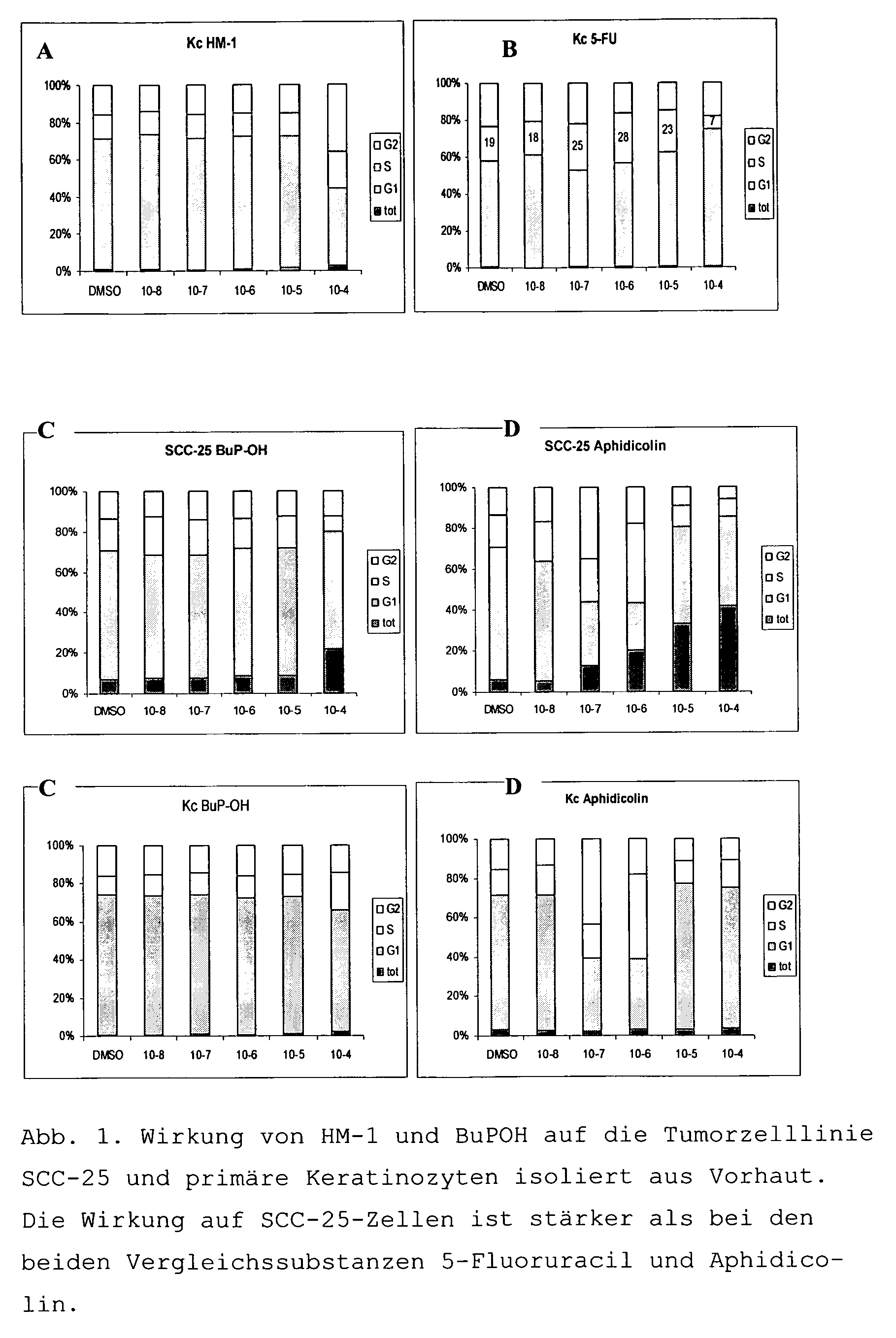

Als besonderer Vorteil von BuP-OH ist der besonders starke antiproliferative Effekt auf Tumorzellen (SCC-25) zu nennen, der den von HM-1 und HM-1-Oxim übertrifft (Abb. 2).Für Tumorzellen besteht eine gewisse Selektivität, die Wirkung ist stärker als auf primäre Keratinozyten und HaCaT-Zellen (Abb. 3, Tab. 1).

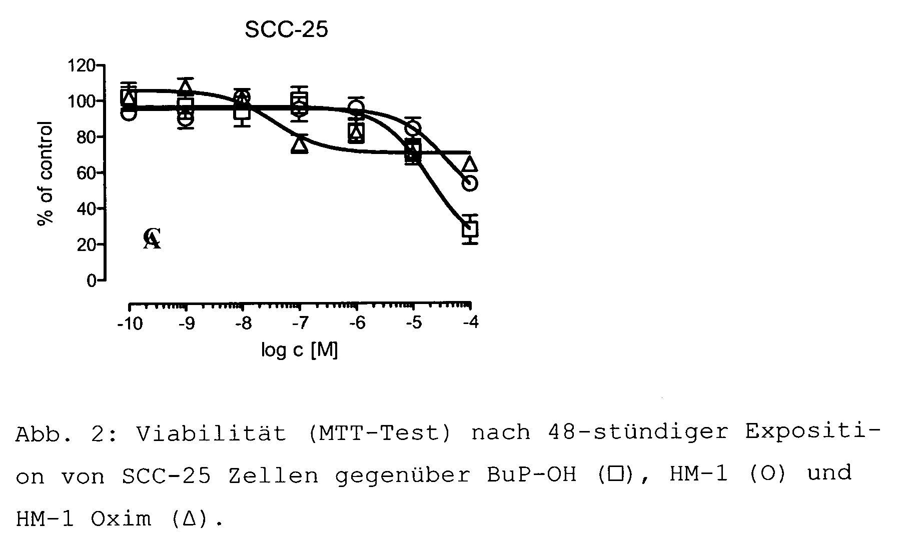

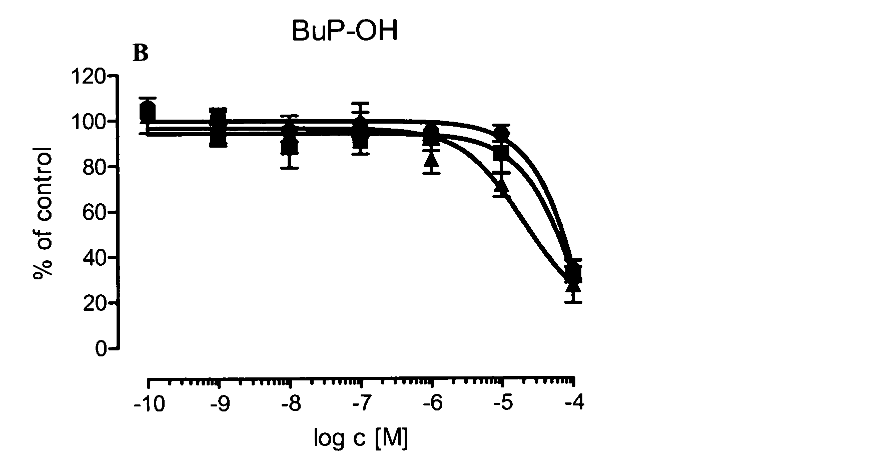

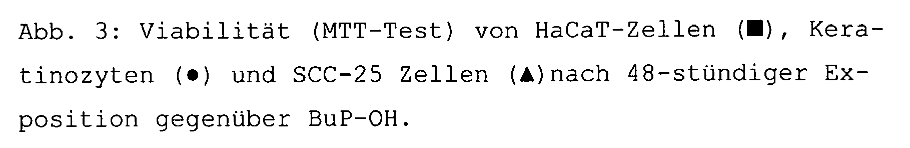

**Tab. 1: -lg IC₅₀ Werte [M] ± SEM ermittelt aus dem MTT-Test (48 h Stimulation). Die maximale Inhibition wurde berechnet als Inhibitionₘₐₓ = 100 - E; E = Aktivität bei 10⁻⁴ M in Prozent. n.d. = not determinable**

| **MTT Test 48 h** | | Primäre Keratin ozyten | | SCC-25-Zellen |
|---|---|---|---|---|
| **Aphidicolin (Positivkontrolle)** | -lg IC₅₀ [M] ± SEM max. Inhibition | 8.25 ± 0.40 | | 6.29 ± 0.29 |
| | | 46 % | | 66 % |
| **5-FU (Positivkontrolle)** | -lg IC₅₀ [M] ± SEM max. Inhibition | 4.98 ± 0.56 | | 5.98 ± 0.28 |
| | | 44 % | | 42 % |
| **BuP-OH** | -lg IC₅₀ [M] ± SEM max. Inhibition | 3.29 ± 1.74 | | 4.69 ± 0.26 |
| | | 66 % | | 73 % |
| **HM-1** | -lg IC₅₀ [M] ± SEM | n.d. | | 4.35 ± 0.43 |
| | max. Inhibition | 24 % | | 47 % |
| **HM-1 Oxim** | -lg IC₅₀ [M] ± SEM max. Inhibition | 7.51 ± 0.79 | | 7.43 ± 0.32 |
| | | 7 % | | 36 % |
| **Foscarnet (Negativkontrolle)** | -lg IC₅₀ [M] ± SEM max. Inhibition | 15,97 n.d. (- | | 5,87 ± 0,24 |
| | | 13 %) | | 9 % |

## Patentansprüche

1. Mittel zur Behandlung von Zellmodifikationen in einem Hautareal, insbesondere ausgewählt aus der Gruppe umfassend maligne Melanome, spinozelluläre Karzinome und/oder Basaliome, das eine Polymerase moduliert,
**dadurch gekennzeichnet, dass**
es ausgewählt ist aus der Gruppe umfassend Verbindungen gemäß den allgemeinen Formeln 1 bis 4:

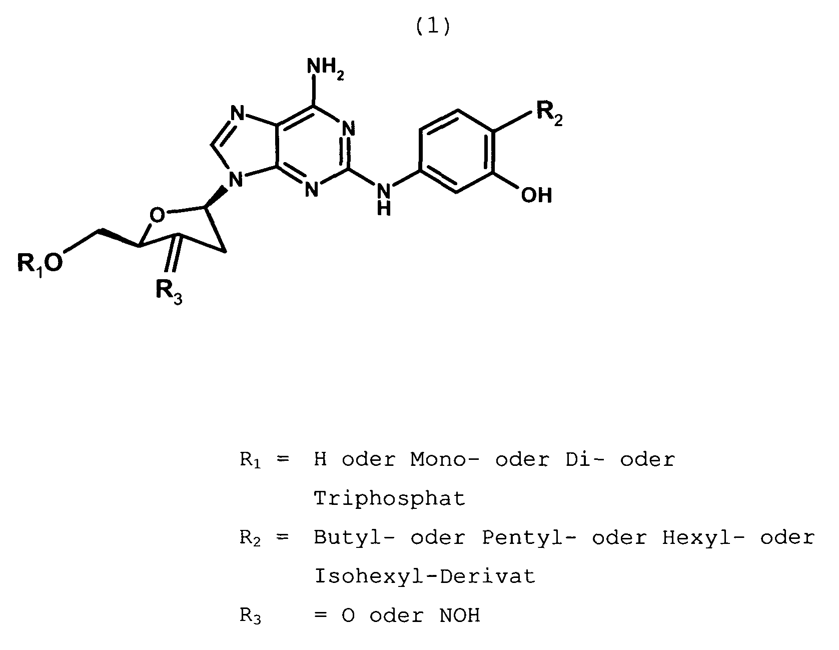

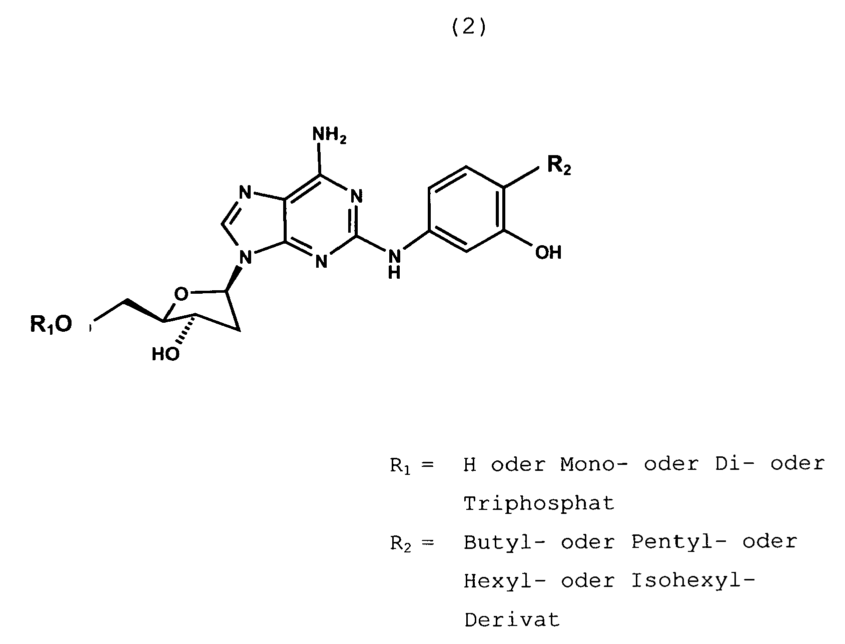

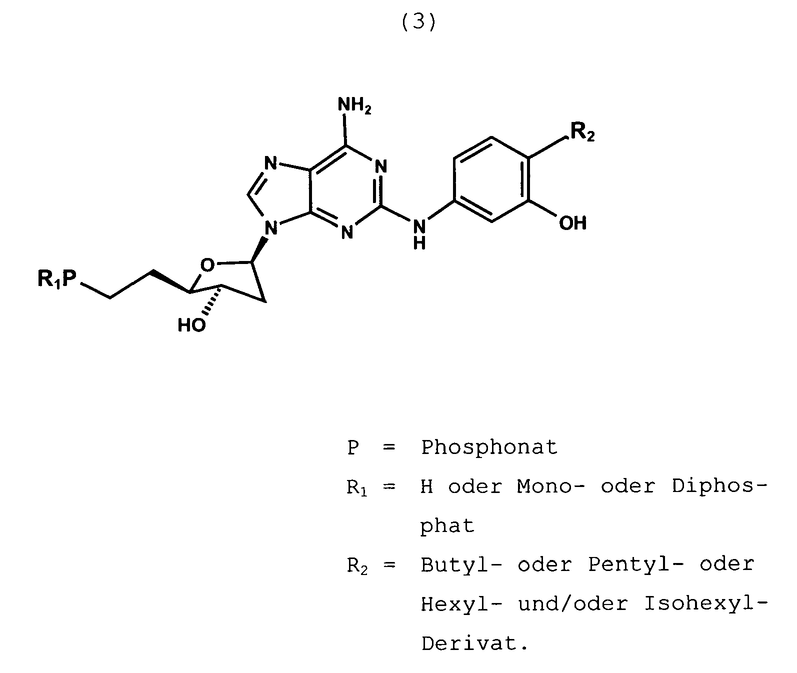

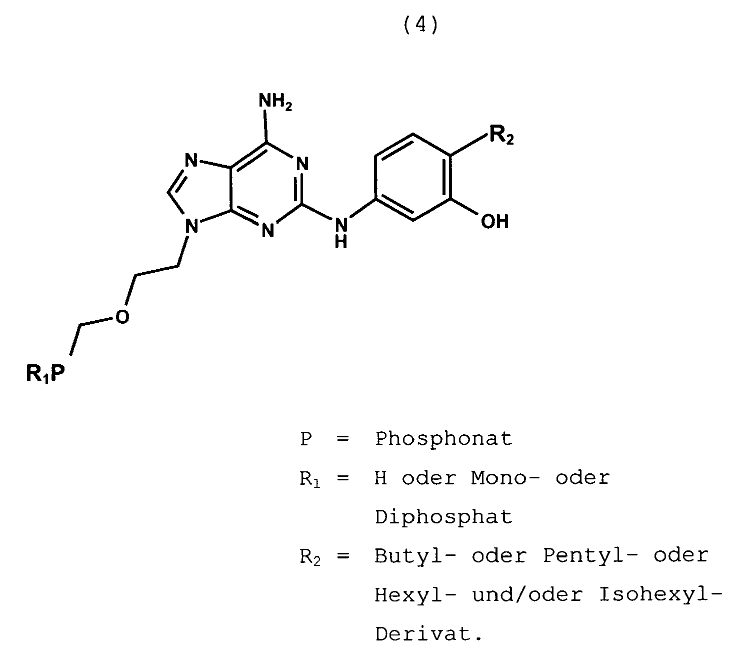

2. Mittel nach Anspruch 1,
**dadurch gekennzeichnet, dass**
es die allgemeine Formel 5 aufweist: 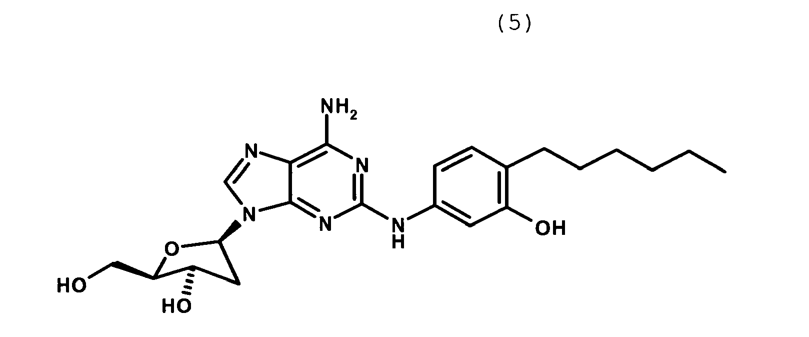 2-(4-Hexyl-3-hydroxyphenylamin) -2'-desoxyadenosin

3. Mittel nach Anspruch 1,
**dadurch gekennzeichnet, dass**
es die allgemeine Formel 6 aufweist: 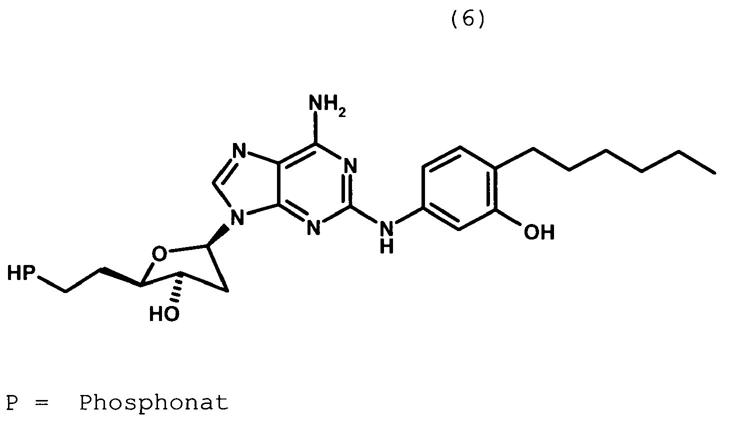 2-(4-Hexyl-3-hydroxyphenylamin)-9-[4-hydroxy-5-(2-phosphonoethyl)tetrahydro-2-furyl]adenin

4. Mittel nach Anspruch 1,
**dadurch gekennzeichnet, dass**
es die allgemeine Formel 7 aufweist: 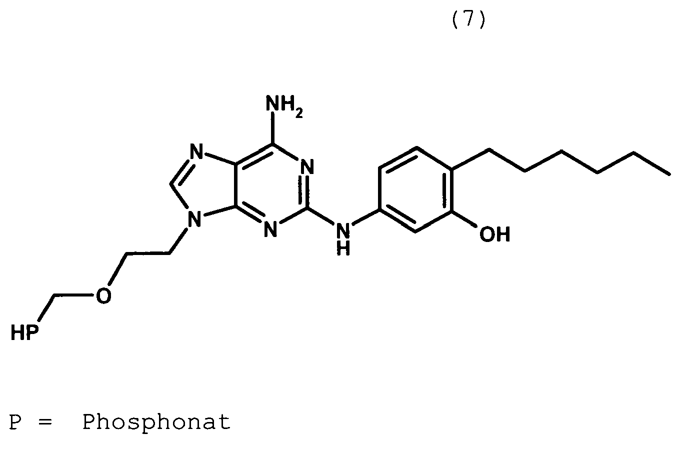 2-(4-Hexyl-3-hydroxyphenylamin)-9-[2-(phosphono-methoxy)ethyl]adenin

5. Mittel nach einem der vorhergehenden Ansprüche,
**dadurch gekennzeichnet, dass**
es funktionsanalog zu einer Verbindung gemäß der Formeln 1 bis 7 ist, ausgewählt aus der Gruppe umfassend 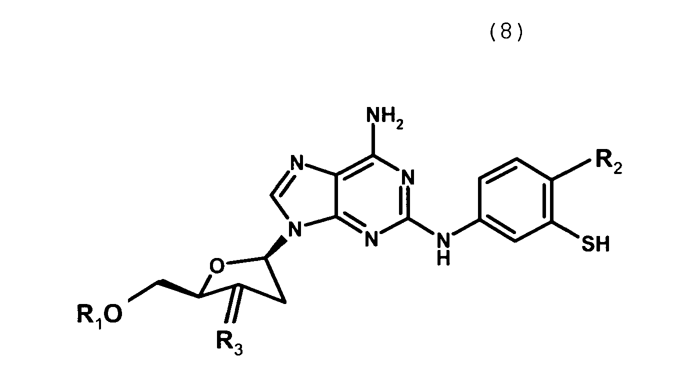 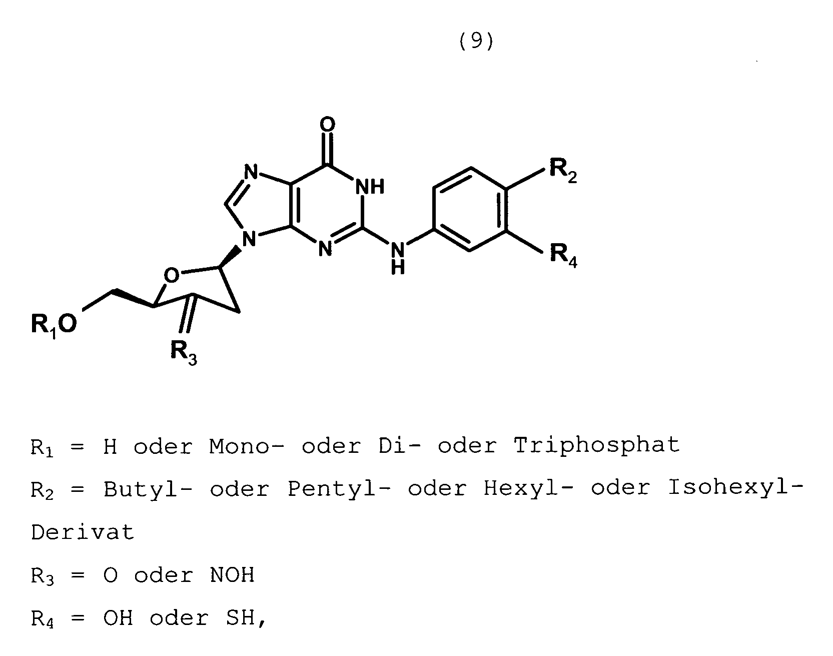 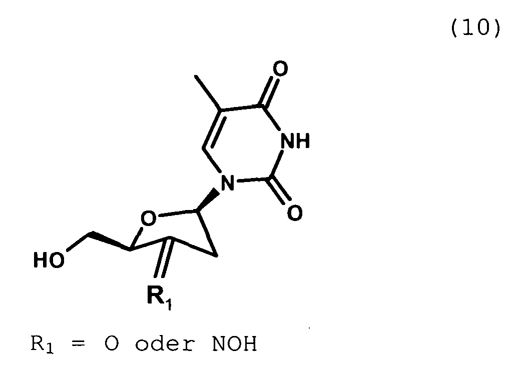 3'-Desoxy-3'-oxothymidin (HM-1)
bzw.
3'-Desoxy-3'-hydroxyliminothymidin,

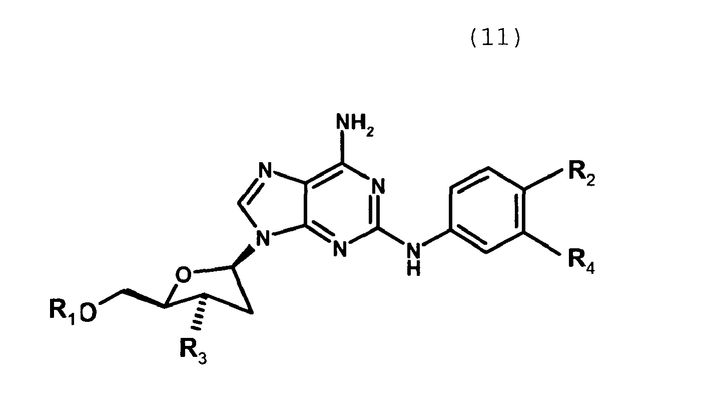

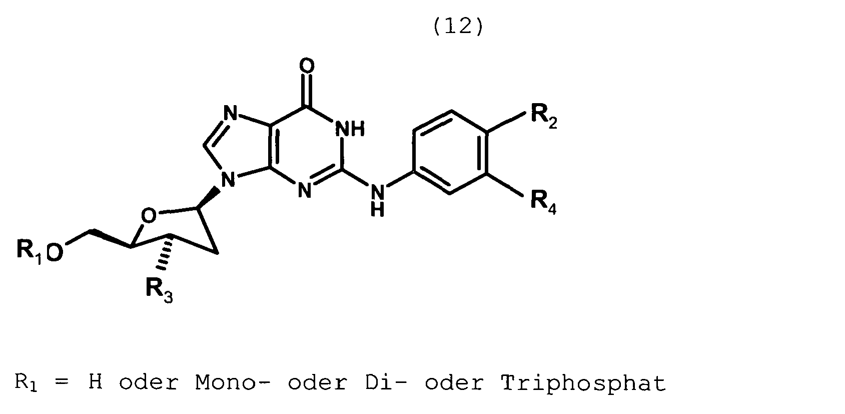

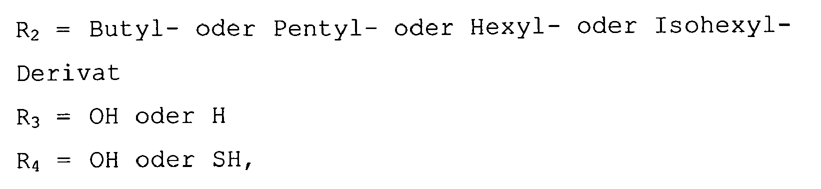

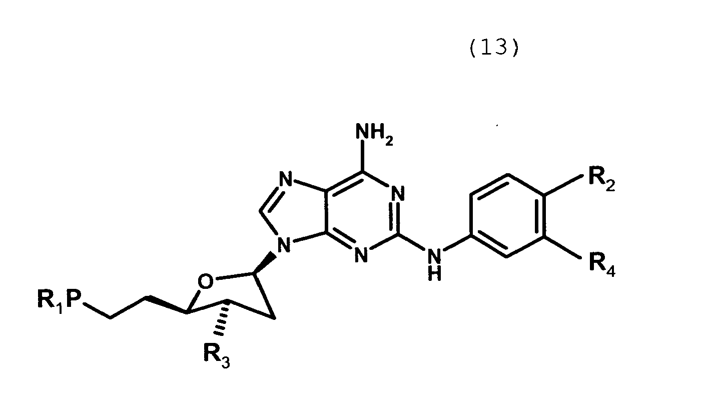

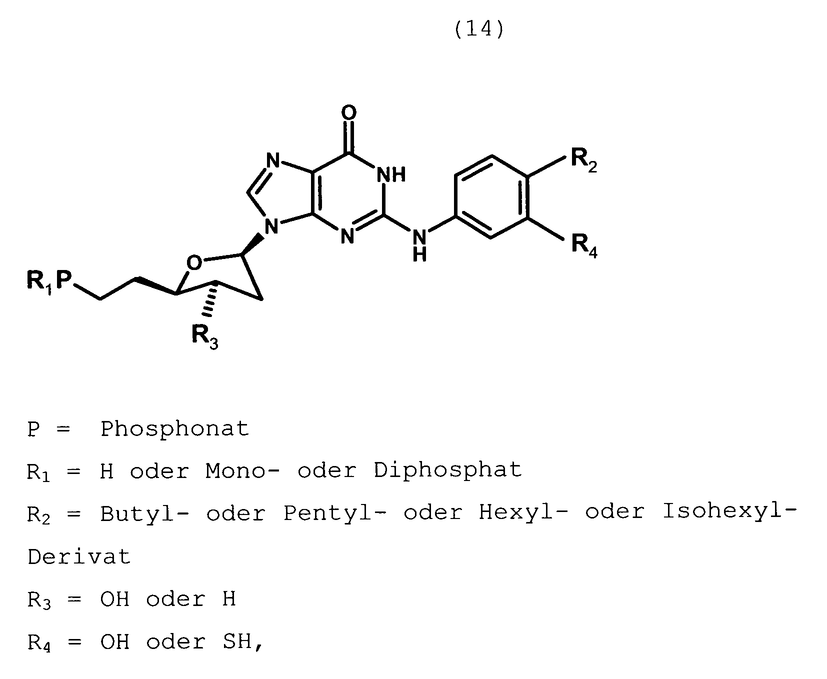

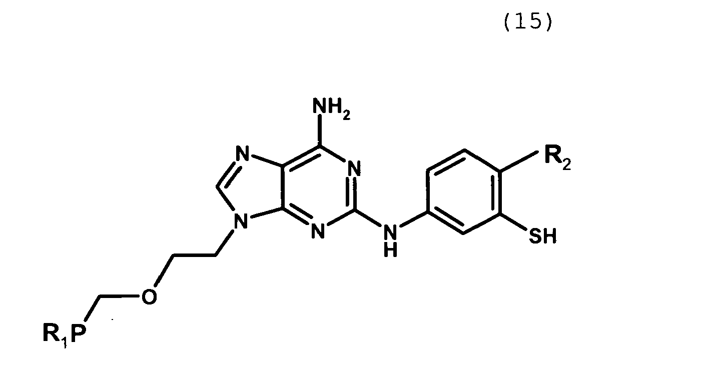

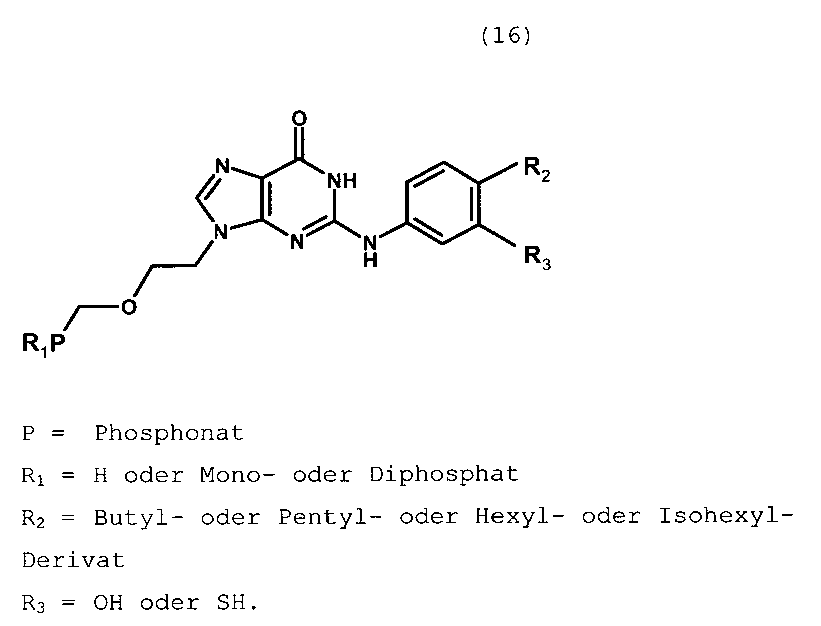

6. Mittel der allgemeinen Formel 1 bis 16 für die Verwendung als Arzneimittel.

7. Pharmazeutisches Mittel, umfassend ein Mittel nach einem der Ansprüche 1 bis 7 und einen pharmazeutisch verträglichen Träger.

8. Pharmazeutisches Mittel nach dem vorhergehenden Anspruch,
**dadurch gekennzeichnet, dass**
der pharmazeutisch verträgliche Träger ausgewählt ist aus der Gruppe umfassend Füllmittel, Streckmittel, Bindemittel, Feuchthaltemittel, Lösungsverzögerer, Sprengmittel, Resorptionsbeschleuniger, Netzmittel, Absorptionsmittel, Gleitmittel und/oder Trägerlipide, insbesondere Liposomen.

9. Kit umfassend ein pharmazeutisches Mittel gemäß der Ansprüche 8 oder 9, zusammen mit einer Information zum Kombinieren der Inhalte des Kits.

10. Verwendung der Mittel nach einem der Ansprüche 1 bis 7, der pharmazeutischen Mittel gemäß einem der Ansprüche 8 oder 9 und des Kits nach Anspruch 10 zur Modifikation einer Polymerase in vitro oder in vivo.

11. Verwendung nach dem vorhergehenden Anspruch,
**dadurch gekennzeichnet, dass**
die Polymerase eine DNA-Polymerase alpha ist.

12. Verwendung nach einem der vorhergehenden Ansprüche,
**dadurch gekennzeichnet, dass**
die Modifikation der Polymerase die Modifikation des Zellwachstums und/oder einer Zellwachstumsstörung umfasst.

13. Verwendung nach dem vorhergehenden Anspruch,
**dadurch gekennzeichnet, dass**
die Zellwachstumsstörung eine Zellproliferation darstellt.

14. Verwendung nach dem vorhergehenden Anspruch,
**dadurch gekennzeichnet, dass**
die Zellproliferation ausgewählt ist aus der Gruppe umfassend Hautkrebs mit Ursprung im Hautepithel, bevorzugt ein Basaliom oder Spinaliom, Hautkrebs der Pigmentzellen, Hautkrebs der Immunzellen, Fibrosarkome, Schweißdrüsenkarzinome, Talgdrüsenkarzinome, Angiosarkome, Myosarkome und/oder Merkelzellkarzinome.

15. Verwendung nach dem vorhergehenden Anspruch,
**dadurch gekennzeichnet, dass**
das Basaliom knotiges, solides Basaliom, ein oberflächliches Basaliom, ein pigmentiertes Basaliom, ein sklerosierend wachsendes Basaliom, ein exulzierend wachsendes Basaliom und/oder ein destruierend wachsendes Basaliom ist.

16. Verwendung nach einem der vorhergehenden Ansprüche,
**dadurch gekennzeichnet, dass**
die Zellwachstumsstörung eine Keratose ist.

17. Verwendung nach dem vorhergehenden Anspruch,
**dadurch gekennzeichnet, dass**
die Keratose ausgewählt ist aus der Gruppe umfassend seborrhoische Keratose, aktinische Keratose, Altersflecken, Pigmentflecken und/oder Lentigo solaris.

18. Verwendung nach einem der vorhergehenden Ansprüche,
**dadurch gekennzeichnet, dass**
die Tumorerkrankung ein Karzinom, ein Sarkom, ein neuroendokriner Tumor, ein hämoonkologischer Tumor, ein dysontogenetischer Tumor und/oder ein Mischtumor ist.

19. Verwendung nach einem der vorhergehenden Ansprüche,
**dadurch gekennzeichnet, dass**
das Mittel in Form einer Lösung, Emulsion, Suspension, Salbe, eines Balsams, Öls, Gels, Schaums, Augenbalsams, Augengels, Zäpfchens, Sprays, Pflasters, Sticks oder Stifts, in flüssiger Form, in Form von künstlichen Tränen, als thermoreversibles Gel (flüssig anzuwenden) und/oder in Form einer Creme vorliegt.

20. Verwendung nach einem der vorhergehenden Ansprüche,
**dadurch gekennzeichnet, dass**
das pharmazeutische Mittel gemäß einem der Ansprüche 10 oder 11 als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und angewendet wird.

21. Verwendung nach dem vorhergehenden Anspruch 5,
**dadurch gekennzeichnet, dass**
die Salbe ausgewählt ist aus der Gruppe umfassend Unguentum leniens (Kühlsalbe), Unguentum emulsificans (Hydrophile Salbe), Unguentum emulsificans aquosum (wasserhaltige hydrophile Salbe), Unguentum cetomacrogolis, Unguentum cetylicum cum aqua (Cetylsalbe), Unguentum Alcoholum Lanae bzw. Unguentum adeps lanae (Wollwachsalkoholsalbe, Eucerin) Unguentum molle (weiche Salbe) und/oder Unguentum Zinci (Zinksalbe).

22. Verwendung nach einem der vorhergehenden Ansprüche,
**dadurch gekennzeichnet, dass**
ein pharmazeutisches Mittel gemäß einem der Ansprüche 10 oder 11 in einer Konzentration von 0,1 bis 99,5, bevorzugt von 0,5 bis 95,0, besonders bevorzugt von 20,0 bis 80,0 Gew.-% in einer Zubereitung vorliegt.

23. Verwendung nach einem der vorhergehenden Ansprüche,
**dadurch gekennzeichnet, dass**
ein pharmazeutisches Mittel gemäß einem der Ansprüche 10 oder 11 in Gesamtmengen von 0,05 bis 500 mg pro kg, bevorzugt von 5 bis 100 mg pro kg Körpergewicht, je 24 Stunden eingesetzt wird.

24. Verwendung nach einem der vorhergehenden Ansprüche,
**dadurch gekennzeichnet, dass**
die Zubereitung oral, subkutan, intravenös, intramuskulär, intraperitoneal und/oder topisch gesetzt wird.

25. Verwendung nach einem der vorhergehenden Ansprüche zur Sekundärprophylaxe von Tumoren.

26. Verwendung der Verbindungen gemäß der Formeln (1) bis (8) als Leitstruktur für die Entwicklung von Polymerasehemmern.
